(19) **Europäisches Patentamt / European Patent Office / Office européen des brevets**

(11) **EP 4 049 024 B1**

(12) # FASCICULE DE BREVET EUROPEEN

(45) Date de publication et mention de la délivrance du brevet:
**12.06.2024 Bulletin 2024/24**

(21) Numéro de dépôt: **20807821.2**

(22) Date de dépôt: **21.10.2020**

(51) Classification Internationale des Brevets (IPC):
*G01N 33/00* *(2006.01)* *B60H 1/00* *(2006.01)*

(52) Classification Coopérative des Brevets (CPC):
**G01N 33/0004;** B60H 1/00785

(86) Numéro de dépôt international:
**PCT/FR2020/051903**

(87) Numéro de publication internationale:
**WO 2021/079064 (29.04.2021 Gazette 2021/17)**

(54) **PROCÉDÉ DE DÉTERMINATION D'UNE HUMIDITÉ RELATIVE DE L'AIR DANS UN HABITACLE DE VÉHICULE AUTOMOBILE**

VERFAHREN ZUM BESTIMMEN DER RELATIVEN LUFTFEUCHTIGKEIT IN EINEM KRAFTFAHRZEUGINNENRAUM

METHOD FOR DETERMINING A RELATIVE HUMIDITY OF THE AIR IN A MOTOR VEHICLE PASSENGER COMPARTMENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.10.2019 FR 1911901**

(43) Date de publication de la demande:
**31.08.2022 Bulletin 2022/35**

(73) Titulaire: **VALEO SYSTEMES THERMIQUES**
**78320 Le Mesnil Saint-Denis (FR)**

(72) Inventeurs:
• **HALLER, Régine**
**78320 LE MESNIL SAINT-DENIS (FR)**
• **MARTINELL, Amanda**
**78320 LE MESNIL SAINT-DENIS (FR)**

(74) Mandataire: **Valeo Systèmes Thermiques**
**Service Propriété Intellectuelle**
**8, rue Louis Lormand**
**La Verrière**
**78320 Le Mesnil Saint Denis (FR)**

(56) Documents cités:
**FR-A1- 3 066 598 US-A- 4 920 755**

EP 4 049 024 B1

**Description**

**Domaine technique et technique antérieure**

La présente divulgation concerne un procédé de détermination d'une humidité relative de l'air dans un habitacle de véhicule automobile.

De nos jours, les véhicules automobiles sont équipés de nombreux capteurs, qui mesurent divers paramètres, afin de garantir un confort toujours amélioré des passagers. Ces capteurs permettent en particulier un ajustement de la température et de l'humidité de l'air dans l'habitacle, ainsi qu'une dépollution de l'air que les passagers sont amenés à respirer. Le document US4920755 décrit par exemple un procédé de détermination d'une humidité relative de l'air dans un habitacle de véhicule automobile où la température et l'humidité relative de l'air mesurées dans un capteur placé sur le pare-brise du véhicule sont employées pour déterminer l'humidité absolue de l'air.

Néanmoins, ces capteurs doivent être associés à des traitements électroniques des données recueillies, ce qui nécessite des ressources électroniques relativement complexes dont il faut prévoir l'implantation dans le véhicule, alors même que les contraintes spatiales sont plus fortes dans un véhicule électrique.

**Résumé**

La présente divulgation vient améliorer la situation.

A cet effet, l'invention a pour objet un procédé de détermination d'une humidité relative de l'air dans un habitacle de véhicule automobile, comprenant les étapes suivantes : détermination d'un paramètre reflétant une humidité spécifique de l'air à partir d'une humidité relative de l'air et une température de l'air dans un capteur intégrant au moins un capteur de température et un capteur d'humidité relative, de préférence un capteur de matière particulaire, et calcul de l'humidité relative de l'air dans ledit l'habitacle du véhicule automobile à partir dudit paramètre et d'une température de l'air dans l'habitacle.

Ainsi, le procédé selon la présente invention permet que le capteur de matière particulaire, en plus de sa fonction première, assure une fonction additionnelle de détermination de l'humidité relative de l'air dans l'habitacle, ce qui évite d'avoir recours à un capteur supplémentaire.

Selon l'invention, ledit paramètre est lié à une pression partielle de l'eau dans l'air par des lois de la thermodynamique.

Selon un autre aspect, au cours de l'étape de détermination de la pression partielle de l'eau dans l'air, on détermine une pression de vapeur saturante à la température de l'air dans le capteur de matière particulaire par une formule thermodynamique de Bertrand-Dupré, de sorte à en déduire la valeur de la pression partielle de l'eau dans l'air.

Selon un autre aspect, au cours de l'étape de détermination de l'humidité relative de l'air dans .l'habitacle du véhicule, on détermine une pression de vapeur saturante à la température de l'air dans l'habitacle par une formule thermodynamique de Bertrand-Dupré, puis on calcule le rapport de la pression partielle de l'eau dans l'air et de ladite pression de vapeur saturante à la température de l'air dans l'habitacle.

Selon un autre aspect, le procédé comprend une étape de détermination de la température de l'air dans l'habitacle du véhicule automobile.

Selon un autre aspect, la température de l'air dans l'habitacle est mesurée par un capteur de température dans l'habitacle.

Selon un autre aspect, la température de l'air dans l'habitacle ($T2$) est estimée à partir de la température de l'air dans le capteur de matière particulaire ($T1$) selon la formule suivante : $T2 = T1 + \Delta$, où $\Delta$ est une constante déterminée expérimentalement.

Selon un autre aspect, la température de l'air dans l'habitacle est estimée à partir de la température de l'air dans le capteur de matière particulaire et à partir d'une puissance dissipée par des composants électroniques d'un circuit électrique disposés dans le capteur de matière particulaire.

Selon un autre aspect, la puissance dissipée est soit mesurée soit connue à partir d'une valeur de tension dans ledit circuit électrique.

L'invention a également pour objet un dispositif pour la mise en oeuvre du tel que décrit précédemment, comprenant un capteur de matière particulaire munie d'un capteur de température de l'air dans ledit capteur et un capteur d'humidité relative de l'air dans ledit capteur.

Selon un autre aspect, il est proposé un programme informatique comportant des instructions pour la mise en oeuvre de tout ou partie du procédé tel que défini précédemment lorsque ce programme est exécuté par un processeur.

Selon un autre aspect, il est proposé un support d'enregistrement non transitoire, lisible par un ordinateur, sur lequel est enregistré un tel programme.

## Brève description des dessins

D'autres caractéristiques, détails et avantages apparaîtront à la lecture de la description détaillée ci-après, et à l'analyse des dessins annexés, sur lesquels :

**Fig. 1**

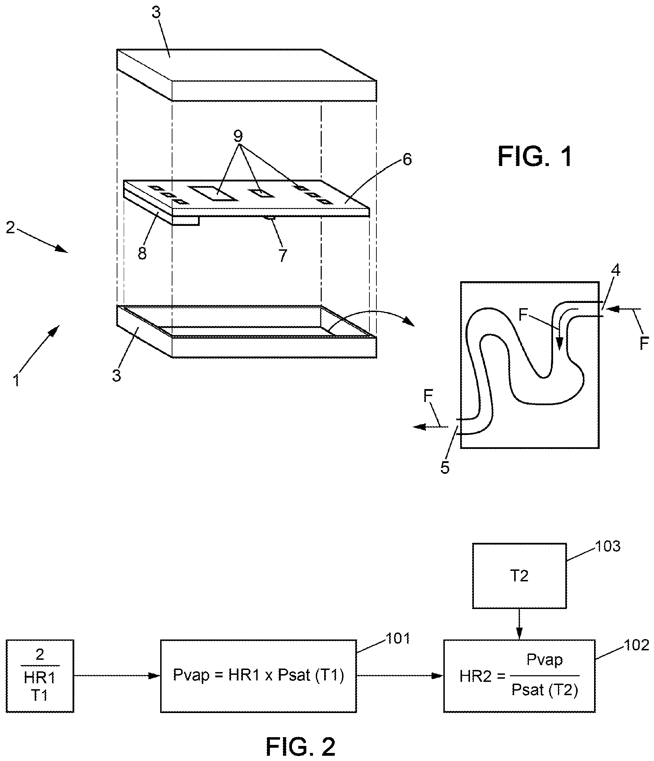

[Fig. 1] illustre partiellement un capteur de matière particulaire pour la mise en oeuvre d'un procédé de détermination de l'humidité relative de l'air dans un habitacle d'un véhicule automobile équipé dudit capteur.

**Fig. 2**
[Fig. 2] illustre un ordinogramme du procédé mis en oeuvre par le capteur de la figure 1 selon un premier mode de réalisation.

**Fig. 3**

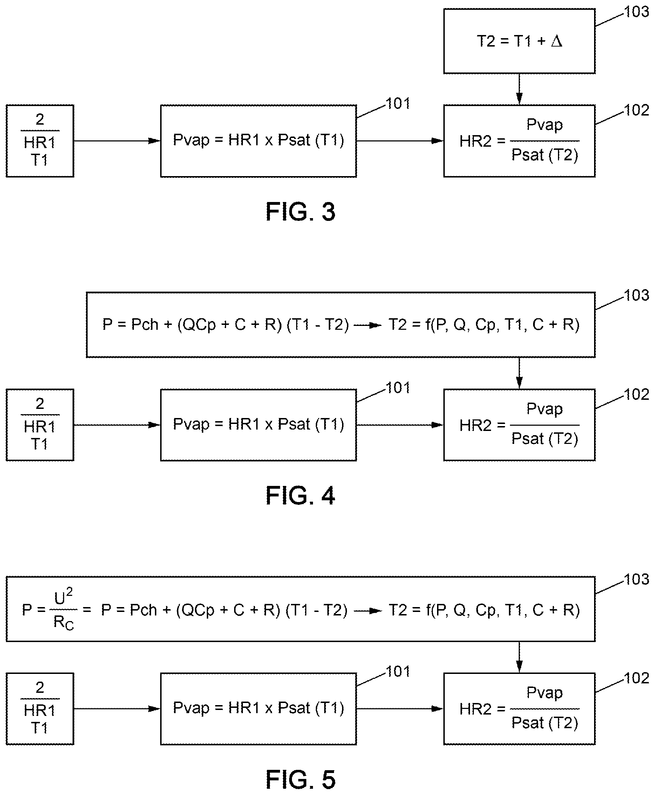

[Fig. 3] illustre un ordinogramme du procédé de la figure 1 selon un deuxième mode de réalisation.

**Fig. 4**
[Fig. 4] illustre un ordinogramme du procédé de la figure 1 selon un troisième mode de réalisation.

**Fig. 5**
[Fig. 5] illustre un ordinogramme du procédé de la figure 1 selon un quatrième mode de réalisation.

## Description des modes de réalisation

Les dessins et la description ci-après contiennent, pour l'essentiel, des éléments de caractère certain. Ils pourront donc non seulement servir à mieux faire comprendre la présente divulgation, mais aussi contribuer à sa définition, le cas échéant.

La présente divulgation a pour objet un procédé 100 de détermination d'une humidité relative de l'air, mis en oeuvre par un dispositif 1 comprenant un capteur de matière particulaire 2, visible sur la figure 1.

Le capteur de matière particulaire 2 fait de préférence partie d'un système de qualité d'air d'un véhicule automobile permettant une dépollution de l'air destiné à alimenter l'habitacle du véhicule automobile. Ce système peut être soit intégré à un dispositif de chauffage, ventilation et/ou climatisation du véhicule automobile, soit un module spécialement dédié. Dans un cas comme dans l'autre, le système peut comprendre avantageusement un purificateur d'air, tel qu'un filtre et/ou un ioniseur, permettant d'assainir le flux d'air qui le parcourt avant de pénétrer dans l'habitacle du véhicule. Il peut également être intégré à un système d'information du passager par une visualisation ou un système d'information plus globale par transmission des données à des serveurs informatiques (nuage) externes à la voiture.

Comme illustré sur la figure 1, le capteur de matière particulaire comprend une enveloppe 3 délimitant un espace interne dans lequel est dessiné un chemin fluidique qu'un flux d'air F emprunte entre une entrée d'air 4 du capteur 2 et une sortie d'air 5 du capteur 2.

Le capteur 2 comprend également des éléments optiques (non illustrés) pour détecter et mesurer une concentration de matière particulaire transportée par le flux d'air F.

Par matière particulaire, on entend toute particule de dimension suffisamment faible pour être transportée par l'air et être inhalée. Les particules peuvent être solides, liquides, ou un mélange de solides et liquides. Par exemple, le diamètre des particules est compris entre 0,01 $\mu$m et 10 $\mu$m. Par exemple, la matière particulaire comprend un mélange de spores, de pollen, de fumée de cigarettes, de carbone ...

Une carte électronique 6 permet une analyse de la mesure effectuée optiquement.

Comme il ressort de la figure 1, la carte électronique 6 porte notamment un capteur 7 d'humidité relative du flux d'air F et un élément de chauffage 8.

La carte électronique porte également d'autres composants électroniques 9 formant un circuit électrique (non illustré).

Le capteur de matière particulaire 2 est avantageusement configuré pour que, si l'humidité relative mesurée est supérieure à une valeur seuil, alors l'élément de chauffage 8 est déclenché, ce qui permet d'assurer que l'humidité reste faible dans le capteur (une humidité trop élevée fausserait la mesure optique et risquerait d'encrasser le capteur optique).

Le capteur de matière particulaire 2 comprend également un capteur de température du flux d'air F non représenté. De préférence, c'est une même puce qui mesure la température et l'humidité relative.

Dans la suite de la demande, la température du flux d'air F dans le capteur de matière particulaire 2 est notée T1 et l'humidité relative du flux d'air F notée HR1.

Dans la suite de la demande, la température de l'air dans l'habitacle du véhicule est notée T2 et l'humidité relative HR2.

Le procédé 100 est maintenant décrit en relation avec les figures 2 à 5.

Comme il ressort de ces figures, le procédé 100 comprend une étape 101 de détermination d'un paramètre reflétant une humidité spécifique de l'air à partir de l'humidité relative HR1 et de la température T1, suivie d'une étape 102 de calcul de l'humidité relative de l'air HR2 à partir dudit paramètre et de la température T2.

De préférence, le paramètre est la pression partielle de l'eau dans l'air, notée Pvap.

Par définition, l'humidité relative HR1 et la pression partielle de l'eau dans l'air Pvap sont liées par l'équation suivante :

$$Pvap = HR1 * Psat(T1), (1)$$

Où Psat(T1) est la pression de vapeur saturante à la température T1.

Selon le procédé 100, la pression de vapeur saturante Psat(T1) est déterminée à partir de la formule suivante, dite Dupré-Bertrand :

$$ln\left(\frac{Psat(T1)}{Po}\right) = 40,164 - \frac{6435,7}{T1} - 3,868 lnT1, (2)$$

Où Po est la pression atmosphérique.

Toutefois, d'autres formules thermodynamiques disponibles dans la littérature scientifique reliant la température et la pression de vapeur saturante peuvent être utilisées. Par exemple, on peut également utiliser la formule de Duperray. Des tables peuvent également être utilisées. La formule de Dupré-Bertrand présente l'avantage d'être particulièrement simple.

Ainsi, la connaissance de la valeur de HR1 (par la mesure) et de la valeur de la pression de vapeur saturante Psat(T1) permet la détermination de la pression partielle de l'eau dans l'air, suivant l'équation (1).

Selon le procédé 100, la pression de vapeur saturante Psat(T2) est déterminée à partir de la formule Dupré-Bertrand :

$$ln\left(\frac{Psat(T2)}{Po}\right) = 40,164 - \frac{6435,7}{T2} - 3,868 lnT2. (3)$$

Selon le procédé 100, l'humidité relative HR2 est déterminée une fois connues la pression partielle de l'eau dans l'air Pvap et la pression de vapeur saturante à la température T2, Psat(T2) :

$$HR2 = \frac{Pvap}{Psat(T2)}. (4)$$

Ainsi, grâce au capteur de matière particulaire 2, il est possible de remonter à la valeur de l'humidité relative dans l'habitacle HR2, ce qui évite d'équiper l'habitacle d'un capteur d'humidité dédié.

Comme il ressort des figures 2 à 5, le procédé 100 comprend également une étape 103 de détermination de la température T2.

Selon le mode de réalisation de la figure 2, la température T2 est mesurée par un capteur dédié (non illustré) positionné dans l'habitacle du véhicule.

Selon le mode de réalisation de la figure 3, la température T2 est estimée à partir de la température T1 selon la formule suivante :

$$T2 = T1 + \Delta, (5)$$

où $\Delta$ est une constante, positive ou négative, déterminée expérimentalement, par exemple au cours d'un test de mesure de la différence de température entre l'habitacle et le capteur 2.

Par exemple, on a déterminé expérimentalement que $\Delta$=-12°C, selon une incertitude de mesure de 0,5°C, pour un capteur PM2,5.

Selon le mode de réalisation de la figure 4, la température T2 est estimée à partir de la température T1 et à partir d'une puissance P dissipée par les composants 9 portés par la carte électronique 6.

Avantageusement, la puissance P est définie par l'équation suivante :

$$P = QCp(T1 - T2) + C(T1 - T2) + R(T1 - T2) + Pch, (6)$$

Où les paramètres sont les suivants :

- $QCp(T1 - T2)$ correspond à la puissance de flux convectif extrait du capteur 2, qui traverse le capteur 2, le coefficient QCp représentant le produit du débit d'air F traversant le capteur 2 par la capacité calorifique,
- $C(T1 - T2)$ correspond à la puissance d'échange thermique entre intérieur et extérieur du capteur 2, autrement dit, il s'agit de la puissance échangée par conduction et convection avec l'extérieur, C représentant la conductance de l'enveloppe du capteur 2,
- $R(T1 - T2)$ correspond à une linéarisation de l'énergie rayonnée par l'enveloppe du capteur 2, et
- Pch est la puissance thermique fournie par la dissipation des composants électroniques et par l'élément de chauffage 8,
- la somme des coefficients C et R, C+R, est avantageusement déterminée expérimentalement. Par exemple, un test a donné les valeurs ci-après :
- P compris entre 0,5 W et 1W,
- QCp compris entre 0,02 et 0,05 W/°C, par exemple 0,04 W/°C, et
- l'échauffement de l'air T1-T2 de l'ordre de 5°C.

Dans ce cas, le coefficient C+R est de l'ordre de 0,2 W.

Ainsi, la température T2 est déterminée par l'équation 6 comme une fonction de P, Q, Cp, T1, C+R.

Selon le mode de réalisation de la figure 5, la puissance P dissipée est déterminée par le biais d'une tension U du circuit électrique selon l'équation suivante :

$$P = \frac{U^2}{Re} (7),$$

Où Re est la résistance équivalente du circuit électrique.

Une fois la puissance dissipée déterminée, la température T2 est estimée à partir de l'équation 6, comme expliqué en relation avec la figure 3.

Ainsi, le procédé 100 permet, de façon complètement inattendue, de déterminer l'humidité relative HR2 de l'air dans l'habitacle à partir du capteur de matière particulaire 2, ce qui limite le nombre de capteurs dont le véhicule automobile doit être équipé.

**Revendications**

1. Procédé de détermination d'une humidité relative de l'air dans un habitacle de véhicule automobile, comprenant les étapes suivantes :

   - détermination (101) d'un paramètre reflétant une humidité spécifique de l'air à partir d'une humidité relative (HR1) de l'air et une température (T1) de l'air dans un capteur de matière particulaire (2) intégrant au moins un capteur de température et un capteur (7) d'humidité relative,
   - calcul (102) de l'humidité relative de l'air (HR2) dans ledit l'habitacle du véhicule automobile à partir dudit paramètre et d'une température (T2) de l'air dans l'habitacle, ledit paramètre étant une pression partielle de l'eau dans l'air (Pvap).

2. Procédé selon la revendication 1, dans lequel, au cours de l'étape de détermination de la pression partielle de l'eau dans l'air (Pvap), on détermine une pression de vapeur saturante (Psat(T1)) à la température (T1) de l'air dans le capteur de matière particulaire (2) par une formule thermodynamique de Bertrand-Dupré, de sorte à en déduire la valeur de la pression partielle de l'eau dans l'air (Pvap).

3. Procédé selon la revendication précédente, dans lequel, au cours de l'étape de détermination de l'humidité relative de l'air (HR2) dans l'habitacle du véhicule, on détermine une pression de vapeur saturante (Psat(T2)) à la température (T2) de l'air dans l'habitacle par une formule thermodynamique de Bertrand-Dupré, puis on calcule le rapport de la pression partielle de l'eau dans l'air (Pvap) et de ladite pression de vapeur saturante à la température de l'air dans l'habitacle (Psat(T2)).

**4.** Procédé selon l'une des revendications précédentes, comprenant une étape de détermination de la température (T2) de l'air dans l'habitacle du véhicule automobile.

**5.** Procédé selon la revendication précédente, dans lequel la température de l'air dans l'habitacle (T2) est mesurée par un capteur de température dans l'habitacle.

**6.** Procédé selon la revendication 4, dans lequel la température de l'air dans l'habitacle (T2) est estimée à partir de la température de l'air dans le capteur de matière particulaire (T1) selon la formule suivante : $T2 = T1 + \Delta$, où $\Delta$ est une constante déterminée expérimentalement.

**7.** Procédé selon la revendication 4, dans lequel la température de l'air dans l'habitacle (T2) est estimée à partir de la température de l'air dans le capteur de matière particulaire (T1) et à partir d'une puissance (P) dissipée par des composants électroniques (9) d'un circuit électrique disposés dans le capteur de matière particulaire (2).

**8.** Procédé selon la revendication précédente, dans lequel la puissance dissipée (P) est soit mesurée soit connue à partir d'une valeur de tension (U) dans ledit circuit électrique.

**9.** Dispositif pour la mise en oeuvre du procédé selon l'une des revendications précédentes, comprenant un capteur de matière particulaire (2) muni d'un capteur de température de l'air dans ledit capteur et un capteur (7) d'humidité relative de l'air dans ledit capteur, ledit dispositif comprenant une carte électronique (6) adaptée pour exécuter les étapes du procédé selon l'une des revendications précédentes.


**Patentansprüche**

**1.** Verfahren zur Bestimmung einer relativen Luftfeuchtigkeit in einem Kraftfahrzeuginnenraum, welches die folgenden Schritte umfasst:

- Bestimmung (101) eines Parameters, der eine spezifische Luftfeuchtigkeit widerspiegelt, aus einer relativen Luftfeuchtigkeit (HR1) und einer Temperatur (T1) der Luft in einem Partikelsensor (2), in den mindestens ein Temperatursensor und ein Relativfeuchtigkeitssensor (7) integriert sind,
- Berechnung (102) der relativen Luftfeuchtigkeit (HR2) in dem Kraftfahrzeuginnenraum aus dem Parameter und einer Temperatur (T2) der Luft im Innenraum, wobei der Parameter ein Partialdruck des Wassers in der Luft (Pvap) ist.

**2.** Verfahren nach Anspruch 1, wobei im Schritt der Bestimmung des Partialdrucks des Wassers in der Luft (Pvap) ein Sättigungsdampfdruck (Psat(T1)) bei der Temperatur (T1) der Luft in dem Partikelsensor (2) durch eine thermodynamische Formel von Bertrand-Dupré bestimmt wird, um daraus den Wert des Partialdrucks des Wassers in der Luft (Pvap) abzuleiten.

**3.** Verfahren nach dem vorhergehenden Anspruch, wobei im Schritt der Bestimmung der relativen Luftfeuchtigkeit (HR2) im Fahrzeuginnenraum ein Sättigungsdampfdruck (Psat(T2)) bei der Temperatur (T2) der Luft im Innenraum durch eine thermodynamische Formel von Bertrand-Dupré bestimmt wird und anschließend das Verhältnis des Partialdrucks des Wassers in der Luft (Pvap) und des Sättigungsdampfdrucks bei der Temperatur der Luft im Innenraum (Psat(T2)) berechnet wird.

**4.** Verfahren nach einem der vorhergehenden Ansprüche, welches einen Schritt der Bestimmung der Temperatur (T2) der Luft im Kraftfahrzeuginnenraum umfasst.

**5.** Verfahren nach dem vorhergehenden Anspruch, wobei die Temperatur der Luft im Innenraum (T2) durch einen Temperatursensor im Innenraum gemessen wird.

**6.** Verfahren nach Anspruch 4, wobei die Temperatur der Luft im Innenraum (T2) anhand der Temperatur der Luft im Partikelsensor (T1) gemäß der folgenden Formel geschätzt wird: $T2 = T1 + \Delta$, wobei $\Delta$ eine experimentell bestimmte Konstante ist.

7. Verfahren nach Anspruch 4, wobei die Temperatur der Luft im Innenraum (T2) anhand der Temperatur der Luft im Partikelsensor (T1) und anhand einer Verlustleistung (P) von elektronischen Komponenten (9) einer elektrischen Schaltung, die im Partikelsensor (2) angeordnet sind, geschätzt wird.

8. Verfahren nach dem vorhergehenden Anspruch, wobei die Verlustleistung (P) entweder gemessen wird oder durch einen Spannungswert (U) in der elektrischen Schaltung bekannt ist.

9. Vorrichtung zur Durchführung des Verfahrens nach einem der vorhergehenden Ansprüche, welche einen Partikelsensor (2) umfasst, der mit einem Lufttemperatursensor in dem Sensor und einem Relativfeuchtigkeitssensor (7) für die Luft in dem Sensor ausgestattet ist, wobei die Vorrichtung eine elektronische Platine (6) umfasst, die dazu eingerichtet ist, die Schritte des Verfahrens nach einem der vorhergehenden Ansprüche auszuführen.

**Claims**

1. Method for determining a relative humidity of the air in a motor vehicle passenger compartment, comprising the following steps:

   - determining (101) a parameter reflecting a specific humidity of the air from a relative humidity (HR1) of the air and a temperature (T1) of the air in a particulate matter sensor (2) integrating at least one temperature sensor and a relative humidity sensor (7);
   - calculating (102) the relative humidity of the air (HR2) in said passenger compartment of the motor vehicle from said parameter and a temperature (T2) of the air in the passenger compartment, said parameter being a partial pressure of the water in the air (Pvap).

2. Method according to Claim 1, wherein, during the step of determining the partial pressure of the water in the air (Pvap), a saturation vapour pressure (Psat(T1)) at the temperature (T1) of the air in the particulate matter sensor (2) is determined using a Bertrand-Dupré thermodynamic formula, so as to deduce therefrom the value of the partial pressure of the water in the air (Pvap).

3. Method according to the preceding claim, wherein, during the step of determining the relative humidity of the air (HR2) in the passenger compartment of the vehicle, a saturation vapour pressure (Psat(T2)) at the temperature (T2) of the air in the passenger compartment is determined using a Bertrand-Dupré thermodynamic formula, then the ratio of the partial pressure of the water in the air (Pvap) to said saturation vapour pressure at the temperature of the air in the passenger compartment (Psat(T2)) is calculated.

4. Method according to one of the preceding claims, comprising a step of determining the temperature (T2) of the air in the passenger compartment of the motor vehicle.

5. Method according to the preceding claim, wherein the temperature of the air in the passenger compartment (T2) is measured by a temperature sensor in the passenger compartment.

6. Method according to Claim 4, wherein the temperature of the air in the passenger compartment (T2) is estimated from the temperature of the air in the particulate matter sensor (T1) in accordance with the following formula: $T2 = T1 + \Delta$, where $\Delta$ is an experimentally determined constant.

7. Method according to Claim 4, wherein the temperature of the air in the passenger compartment (T2) is estimated from the temperature of the air in the particulate matter sensor (T1) and from a power (P) dissipated by electronic components (9) of an electrical circuit that are disposed in the particulate matter sensor (2).

8. Method according to the preceding claim, wherein the dissipated power (P) is either measured or known from a value of the voltage (U) in said electrical circuit.

9. Device for implementing the method according to one of the preceding claims, comprising a particulate matter sensor (2) provided with a sensor of the temperature of the air in said sensor and a sensor (7) of the relative humidity of the air in said sensor, said device comprising a circuit board (6) designed to execute the steps of the method according to one of the preceding claims.

**FIG. 1**

$$\frac{2}{HR1} \quad T1$$

$$\xrightarrow{} \quad \boxed{Pvap = HR1 \times Psat\ (T1)} \quad 101$$

$$\xrightarrow{} \quad \boxed{HR2 = \frac{Pvap}{Psat\ (T2)}} \quad 102$$

$$\boxed{T2} \quad 103$$

**FIG. 2**

103

$$T2 = T1 + \Delta$$

$$\dfrac{2}{HR1}$$
$$T1$$

101

$$Pvap = HR1 \times Psat\,(T1)$$

102

$$HR2 = \dfrac{Pvap}{Psat\,(T2)}$$

**FIG. 3**

103

$$P = Pch + (QCp + C + R)\,(T1 - T2) \longrightarrow T2 = f(P, Q, Cp, T1, C + R)$$

$$\dfrac{2}{HR1}$$
$$T1$$

101

$$Pvap = HR1 \times Psat\,(T1)$$

102

$$HR2 = \dfrac{Pvap}{Psat\,(T2)}$$

**FIG. 4**

103

$$P = \dfrac{U^2}{R_C} = P = Pch + (QCp + C + R)\,(T1 - T2) \longrightarrow T2 = f(P, Q, Cp, T1, C + R)$$

$$\dfrac{2}{HR1}$$
$$T1$$

101

$$Pvap = HR1 \times Psat\,(T1)$$

102

$$HR2 = \dfrac{Pvap}{Psat\,(T2)}$$

**FIG. 5**

**EP 4 049 024 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4920755 A